(19) Europäisches Patentamt
European Patent Office
Office européen des brevets

(11) Publication number: **0 127 130**
**A2**

(12) # EUROPEAN PATENT APPLICATION

(21) Application number: 84105900.9

(22) Date of filing: 24.05.84

(51) Int. Cl.³: **A 61 K 45/02**
A 61 K 9/14

(30) Priority: 31.05.83 US 499138

(43) Date of publication of application:
05.12.84 Bulletin 84/49

(84) Designated Contracting States:
AT BE CH DE FR IT LI NL SE

(71) Applicant: SCHERING CORPORATION
2000 Galloping Hill Road
Kenilworth, New Jersey 07033(US)

(72) Inventor: Zupon, Michael Anton
15 Hillside Avenue
Madison New Jersey 07940(US)

(72) Inventor: Sequeira, Joel August
18 Stuyvesant Oval
New York New York 10009(US)

(72) Inventor: Kirschner, Alan Steven
15 Rutledge Court
East Brunswick New Jersey 08816(US)

(72) Inventor: Yuen, Pui-Ho
278 Savoy Avenue
Edison New Jersey 08820(US)

(74) Representative: Antony, Fritz, Dr. et al,
c/o Scherico Ltd. P.O. Box 601 Töpferstrasse 5
CH-6002 Lucerne(CH)

(54) **Interferon gel formulations.**

(57) A kit for formulating and dispensing an alpha-type interferon gel composition comprising a) a vial having an open end aseptically sealed and containing about $1 \times 10^4$ to $5 \times 10^8$ International Units of lyophilized alpha-type interferon formulation prepared from an alpha-type interferon having a specific activity of at least $5 \times 10^7$ International Units/mg. total protein; and b) a tube having flexible walls and sealing means at its open end and containing a dermatologically acceptable vehicle which contains a compatible preservative and a sufficient amount of polyoxyethylene polyoxypropylene block polymer for the vehicle to be liquid at about 15°C. and below and which togethr with the lyophilized alpha-type interferon gels at 15°C. and above; and methods for preparing said gel using said kit.

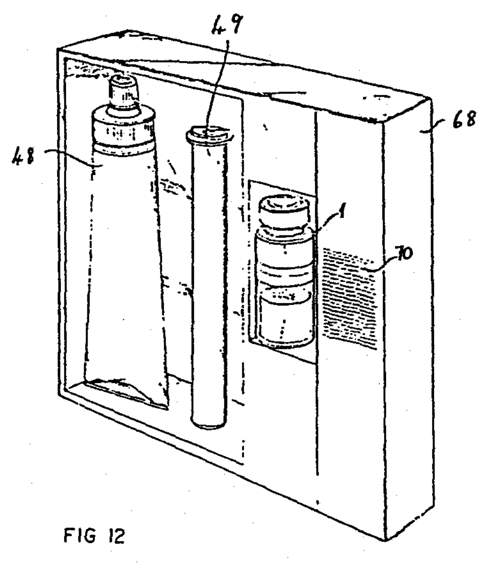


FIG 12

EP 0 127 130 A2

Croydon Printing Company Ltd

# INTERFERON GEL FORMULATIONS

This invention relates to a kit with which one may prepare and topically administer a lyophilized alpha-type interferon gel.

It is widely believed that alpha-type interferons, applied topically, have great potential as drugs for the treatment of a wide variety of disease states and particularly for various types of viral infection, e.g. herpes. Alpha-type interferons are unstable in aqueous solution over an extended period of time and, in order to minimize decomposition of the alpha-type interferon during storage, the aqueous solution is lyophilized. The lyophilized alpha-type interferon is reconstituted with water immediately prior to administration. Topical administration of the reconstituted lyophilized alpha-type interferon has been considered unpractical since the resulting known reconstituted formulations lack sufficient consistency therefor.

We have developed a kit for easy reconstitution of a lyophilized alpha-type interferon as a gel suitable for topical application. Combining the dermatologically acceptable vehicle of this kit with the lyophilized alpha-type interferon powder surprisingly results in an elegant gel formulation. In particular, the present invention pertains to a kit for formulating and dispensing an alpha-type interferon gel composition comprising a) a vial having an open end aseptically sealed and containing about $1 \times 10^4$ to $5 \times 10^8$ International Units of lyophilized alpha-type interferon formulation prepared from an

alpha-type interferon having a specific activity of at least 5 x $10^7$ International Units/mg. total protein; and b) a tube having flexible walls and sealing means at its open end and containing a dermatologically acceptable vehicle which contains a compatible preservative and a sufficient amount of polyoxyethylene polyoxypropylene block polymer for the vehicle to be a liquid at about 15°C. and below and which together with the lyophilized alpha-type interferon gelts at about 15°C. and above.

Preferably the reconstituted alpha-type interferon gel will contain about 1 x $10^4$ to 5 x $10^8$ I.U. of alpha-type interferon per gram of vehicle; i.e. the vial will contain about 1 x $10^4$ to 5 x $10^8$ I.U. of alpha-type interferon per gram of vehicle in the tube.

As used herein, the term "alpha-type interferon" means an interferon exhibiting biological properties similar to those of human leucocyte interferon. It should be noted that a number of human alpha-interferon species are known, usually designated by a numeral after the Greek letter. Also included within the scope of this invention are the so-called alpha hybrid interferons wherein fragments of two native alpha-interferon species are joined (see for instance EPA 51,873). Preferred forms of alpha-interferon for use in the formulations of the present invention are alpha-1 and alpha-2 interferon. Most preferably, the formulations of the present invention employ alpha-2 interferon which may be prepared by recombinant-DNA methods, for example those disclosed by Nagata et al., Nature, 284, 316-320 (1980).

By the term "topical" it is meant that the gel is suitable for vaginal, ophthalmic, rectal and/or dermal administration.

The term "dermatologically acceptable vehicle" means a pharmaceutical vehicle which is substantially non-toxic

and non-irritating to the intended site of administration, e.g. the skin, eyes, or mucous membranes.

The invention also provides a method for preparing an alpha-type interferon gel composition, comprising:

a) dissolving lyophilized alpha-type interferon in a vial by adding a measured amount of distilled water;

b) adding the resulting solution of alpha-type interferon to a tube with flexible walls containing a dermatologically acceptable vehicle maintained at about 15°C. or below, said vehicle containing a compatible preservative and a sufficient amount of polyoxyethylene polyoxypropylene block polymer for the vehicle to be liquid at about 15°C. or below and to gel at above about 15°C; and

c) forming the gel from the said vehicle and alpha-type interferon solution by allowing the contents of the tube to reach a temperature above about 15°C.

The invention further provides a method for preparing an alpha-type interferon gel composition comprising:

a) dissolving lyophilized alpha-type interferon at about 15°C. or below in a measured amount of a dermatologically acceptable vehicle which contains a compatible preservative and a sufficient amount of polyoxyethylene polyoxypropylene block polymer for the vehicle to be liquid at about 15°C. and below and which together with the lyophilized alpha--type interferon gels at 15°C. and above;

b) transferring the reconstituted solution of interferon to a tube with flexible walls; and

c) forming the gel from the said vehicle and alpha-type interferon solution by allowing the contents of the tube to reach a temperature above about 15°C.

For the better understanding of the invention, a preferred embodiment thereof will be described with reference to the accompanying drawings, wherein:

Figure 1 is an isometric view of the aseptically sealed vial found in the kit of the present invention;

Figure 2 is a top view of the vial cover seen along the arrows 2-2 of Figure 1;

Figure 3 is an isometric view of the same vial cover seen along the arrows 3-3 of Figure 1;

Figures 4, 5, 6 and 7 illustrate various views of the vial during different stages of opening;

Figure 8 illustrates the various components of the tube with flexible walls found in the kit of the present invention;

Figure 9 illustrates an applicator adaptable to fit onto the tube with flexible walls and suitable for topical vaginal application of the alpha-type interferon gel formulation;

Figure 9a illustrates an end view of the applicator seen along arrows 9a-9a of Figure 9;

Figure 10 is a partly sectional view of the part 56-56 of Figure 9;

Figure 11 illustrates an alternative vial for the kit of the present invention; and

Figure 12 illustrates a convenient package in which the components of the kit of the present invention may be stored.

Figure 1 shows a vial 1 containing a lyophilized alpha-type interferon formulation 10. Vial 1 has a rubber or plastic stopper 14 extending into the neck 24 of the vial 1 and overfitted with a metallic cap 12.

Figure 2 and Figure 3 illustrate metallic cap 12 seen along arrows 2-2 and 3-3 respectively of Figure 1. Metallic cap 12 include two cuts 32, which join to two curved cuts 34, and also a further cut 28 at the rear of the cap. Curved cuts 34 are separated from a semicircular cut 37 by struts 33.

Cuts 34 and 37 thus define a ring 16 forming a part of and situated inside the top of cap 12. Struts 33 serve as a means for attaching ring 16 to the remainder of the cap 12. Ring 16 serves as an inner pull tab attached to an outer crimp portion 18 of cap 12 by struts 33 and by bridges at the bottom of cuts 32. The struts 33 are easily broken allowing for ready removal of the inner pull tab from the metallic cap 12 during opening. In semicircular cut 37 there is a raised portion 35 of ring 16 which allows for placement of a fingernail or the like under the ring 16 so that it can be used as a pull tab at the time of opening.

Figure 4 illustrates a partially opened cap for the vial 1, revealing metallic lid 20 situated between metallic cap 12 and rubber or plastic stopper 14. The pull tab 16 is removed from the cap 12 by first placing a fingernail or the like under the raised portion 35 of semicircular cut 37. Ring or pull tab 16 is detached from the cap 12 by pulling forward and thus severing the struts 33 and bridges at the bottom of cut 32. Continued pulling on pull tab 16 completely detaches it from the cap 12, leaving the crimped portion of the cap 18; this may easily be removed by forcing the ends at the cuts 32 away from each other and thus opening cut 28.

Figure 5 illustrates the vial of the present invention after the metallic cap 12 has been completely removed. The mouth of the vial, as defined by rim 22, is

still closed by stopper 14 overfitted with the metallic lid 20. The metallic lid 20 may be removed by placing a fingernail or the like between the metallic lid 20 and the stopper 14 and pushing the metallic lid 20 off.

Figure 6 illustrates the vial of the present invention after the metallic lid 20 has been removed. The stopper 14 fits into the mouth of the vial, over the rim 22, and extends downward into the neck 24 of the vial 1. The stopper 14 is removed by placing a finger between the rim 22 and the stopper 14 and pulling the stopper out of the vial.

Figure 7 illustrates a completely opened vial 1, partially filled with a lyophilized alpha-type interferon formulation 10. A measured amount of distilled water may now be added to the vial 1 to dissolve the formulation. If gentle shaking is necessary, stopper 14 may again be placed into the mouth of the vial 1 so as to avoid any loss of the sample.

Figure 8 illustrates the various parts of a second component of the kit which is squeeze tube 48 with flexible walls. The squeeze tube 48 has a neck 45 with outer threads 46 and a wide opening 47. A lid 36 has inner threads (not shown) for attaching to the outer threads 46. Lid 36 also has a dispenser opening 40 formed by a protrusion 42 having outer threads 38. A cap 44 has inner threads (not shown) for attaching to the outer threads 38.

Figure 9 illustrates an applicator 49 (in the extended position) suitable for the vaginal application of the alpha-type interferon gel formulation. Applicator 49 consists of a reservoir member 50 and a plunger 52. The plunger 52 has a stopper 62 and a flange 54. The plunger may be inserted into the reservoir member at rim 63. Plunger 52 serves as a piston within a cylinder bore defined by reservoir 50.

Figure 9A illustrates an end view of the applicator 49 seen along the lines 9a-9a of Figure 9. The reservoir member 50 has inner threads 60 for attaching the applicator to the outer threads 38 of protrusion 42. The inner threads 60 extend upward into the reservoir 50 so as to completely mate with outer threads 38.

Before the reservoir 50 is filled, the applicator 49 must first be attached to the tube 48 by mating the outer threads 38 with the inner threads 60. Plunger 52 is then extended from the reservoir 50 while squeezing tube 48 thus filling the reservoir 50 with the alpha-type interferon gel formulation contained in the tube. After removing the applicator 49 from the squeeze tube 48, the formulation may then be applied as desired by depressing the plunger 52 into the reservoir 50.

Figure 10 is a partly sectional view of the vaginal applicator 49 at 56-56 in Figure 9 and illustrates stopper 61. Stopper 61 is fitted at the end of the plunger 52 and contains ribs 62 and protrusion 64. The protrusion 64 extends from the end of the stopper 61, and is used to facilitate application of the gel formulation. The ribs 62 on stopper 61 are wider than rim 63 of the reservoir 50 and prevent the removal of the plunger 52 in normal use. However, in a preferred embodiment, the walls of the reservoir 50 are sufficiently flexible to allow the plunger 52 to be removed from the reservoir 50 by pulling. This allows the user to readily clean the applicator 49.

Figure 11 illustrates a vial 11 having a neck 24 with outer threads 66. Stopper 14 is placed into the top of the vial. Lid 67 has inner threads (not shown) for mating with the outer threads 66. In this embodiment, the vial 11 is rapidly opened and closed by turning the lid 67 and removing the stopper 14.

Figure 12 illustrates one method of packaging the kit

of the present invention. The tube 48 with flexible walls (together with the applicator 49 if desired) is stored separate from vial 1 in box 68. Optionally, instructions 70 may be provided on the box 68.

We have found that the problem of decomposition of alpha-type interferon when employed in topical formulations can be easily and surprisingly eliminated by employing the kit of the present invention. In particular, we have found that if the lyophilized alpha-type interferon is separately stored and is then added to the pharmaceutical vehicle immediately prior to use the resulting alpha-type interferon formulation will retain sufficient activity to ensure a therapeutic effect.

The kit of the present invention may readily be used to prepare at the time of use a lyophilized alpha-type interferon gel suitable for topical application. For example, tube 48 as supplied with the kit is partially filled with a dermatologically acceptable vehicle which contains a compatible preservative and a sufficient amount of polyoxyethylene polyoxypropylene block polymer for the vehicle to be liquid at about 15°C. or below and to gel at above about 15°C. Lyophilized alpha-type interferon 10 may be dissolved by addition of a measured amount of water to vial 1 which has been opened as described. The dissolved alpha-type interferon formulation 10 can be poured into tube 48 by its wide neck 47 at or below 15°C. Lid 36 (with cap 44) is then attached to tube 48. Tube 48 is gently shaken to ensure homogeneity of the alpha-type interferon and then allowed to reach room temperature to form the gel. The gel may then be conveniently dispensed via dispenser opening 40.

A preferred manner of preparing the lyophilized alpha-type interferon gel formulation using the kit of the present invention comprises first dissolving the lyophilized alpha-type interferon by adding a measured amount of distilled water to the vial containing the lyophilized alpha-type interferon; adding the resulting solution to a tube with flexible walls containing a dermatologically acceptable vehicle maintained at about 15°C. or below; said vehicle containing a compatible preservative and a sufficient amount of polyoxyethylene polyoxypropylene block polymer for the vehicle to be liquid at about 15°C. or below and to gel at above about 15°C.; and forming the gel from the said vehicle and alpha-type interferon solution by allowing the contents of the tube to reach a temperature above about 15°C.

In this way one can prepare a novel pharmaceutical gel formulation comprising $1 \times 10^4$ to $5 \times 10^8$ International Units of an alpha-type interferon formulation prepared from an alpha-type interferon having a specific activity of at least $5 \times 10^7$ International Units/mg. total protein in a dermatologically acceptable vehicle containing a compatible preservative and a sufficient amount of polyoxyethylene polyoxypropylene block polymer for the vehicle to be a liquid at about 15°C. and below and which together with the alpha-type interferon gels at above about 15°C.

In a preferred aspect of the present invention, the tube with flexible walls employed in the kit of the present invention has a wide opening to allow for the easy addition of the reconstituted alpha-type interferon solution. The closure for the tube advantageously carries a dispenser which allows for a small amount of the gel to be removed from the tube upon squeezing while at the same time closing the wide opening.

Preferably, the dermatologically acceptable vehicle employed in the kit will be stored under refrigeration (2°C. to 8°C.) immediately prior to use. At this

temperature the vehicle will be a liquid. Following reconstitution of the lyophilized interferon with the refrigerated vehicle (liquid state), the composition is allowed to achieve room temperature whereupon it forms a gel suitable for topical administration.

The gels employed in the present invention are generally clear in appearance and in addition to the polyoxyethylene polyoxypropylene block polymer may contain other medicinals, appropriate buffers, skin/mucus permeation enhancers, coloring agents and perfumes.

If an opaque gel is desired, i.e. one having a cream-like appearance, 0.1 to 1.0 percent by weight of titanium dioxide may be included in the dermatologically acceptable vehicle and thus be added to the gel.

In a preferred aspect of the present invention a preservative amount of benzyl alcohol is employed in the dermatologically acceptable vehicle. The concentration of benzyl alcohol which is used is known in the art although in the present invention between 0.5% and 3.0% of benzyl alcohol is generally employed. Other known preservatives such as chlorocresol, methyl p-hydroxybenzoate and the like may be employed in the present formulation in place of benzyl alcohol and are equivalent to benzyl alcohol.

In order to enhance the effectiveness of benzyl alcohol as a preservative in the present formulation, it is desirable to maintain the formulation at a pH of 7 or and below, preferably by means of a buffer system. Phosphoric acid/monobasic sodium phosphate is the preferred buffer system although other suitable known buffer systems may equally be used. The individual amounts of phosphoric acid and monobasic sodium phosphate used in the present formulations are generally dependent on the desired pH of the system to be attained.

However, it is preferable not to maintain the pH of the formulation at below pH 2 since such a formulation is not pharmaceutically acceptable.

In a preferred aspect of the present invention, the pH of the dermatologically acceptable vehicle is maintained between pH 3.0 and pH 4.5 by a phosphoric acid/ monobasic sodium phosphate buffer.

The amount of alpha-type interferon contained in the formulations of the present invention is 1 x $10^4$ to 5 x $10^8$ International Units, and such an amount is preferably contained in one gram of reconsituted composition in the form of a gel. Preferably the amount of alpha-type interferon contained in the formulations of the present invention is 1 x $10^6$ to 1 x $10^8$ I.U. per gram of gel.

The lyophilized alpha-type interferon preferably contains an effective amount of glycine to act as a stabilizer, as disclosed in European Patent Application No. 82481A (the disclosure of which is incorporated herein by reference).

In order to avoid possible side effects and to ensure reproducibility of observed therapeutic effects, it is desirable to use alpha-type interferons of high specific activity.

The specific activity of alpha-type interferon used in the formulations of the present invention should be at least 5 x $10^7$ International Units/mg. total protein. Specific activity may be determined by measuring the antiviral activity as compared to the NIH reference standard and by measuring the total protein content using standard methods (e.g. the Lowry method).

An important aspect of the present invention is the use of an appropriate gelling agent in the dermatologically acceptable vehicle. The gelling agent used must allow for the vehicle to be in a liquid state at one temperature, for reconstitution of the lyophilized alpha-type interferon, and in a gel state at room temperature. Additionally, since the gel formulation is to be prepared prior to use by either the pharmacist or an unskilled patient, the gelling agent must be both highly efficient in forming the gel and easy to handle. Gelling agents possessing these qualities are specific poloxamer compounds, polyoxyethylene-polyoxypropylene block polymers, described in detail by Schmolka et al. in U.S. Patent 3,740,421 (incorporated herein by reference) and represented by the following formula:

$$HO(C_2H_4O)_b\ (C_3H_6O)_a\ (C_2H_4O)_b\ H$$

wherein a is an integer such that the hydrophobe represented by $(C_3H_6O)$ has an average molecular weight of from 2250 to 4000 and b is a number from 16 to 360.5 such that the hydrophile represented by $(C_2H_4O)$ constitutes from 10 to 90 weight percent of the polymer. The gels of this invention are composed of from 15 to 90 weight percent polyoxyethylene-polyoxypropylene block polymer. A particularly preferred gelling agent is Poloxamer 407 (Pluronic F-127 available from BASF Wyandotte Corp. of Wyandotte, Michigan) since at appropriate concentrations the gel vehicle is an easily manageable liquid at about 15°C. and below. The lyophilized alpha-type interferon is rapidly and uniformly dissolved in the cooled gel vehicle (in the liquid state) and uniformly mixed by gentle shaking. A preferred aspect of this invention is a kit which allows for a reconstituted solution, e.g., lyophilized alpha-type interferon reconstituted with water, to be added to the cooled gel vehicle (in the liquid state) and to be uniformly mixed by gentle shaking. These simple features facilitate preparation of the formulation when and where it will be used and minimize the likelihood of error.

The following non-limiting examples illustrate the kit of the present invention.

## EXAMPLE 1

Preparation of the dermatologically acceptable vehicle

| Ingredients | | mg./g. |
|---|---|---|
| Poloxamer 407 | | 200.0 |
| Phosphoric Acid NF | | 0.5 |
| Monobasic Sodium Phosphate USP | | 2.94 |
| Benzyl Alcohol NF | | 22.222 |
| Water, Purified USP | q.s. to make | 1.0 g. |

The dermatologically acceptable vehicle may be manufactured using either a hot or a cold method.

a. Hot Method

1. Heat 90% of the purified water to 80-90°C.

2. While gently stirring, slowly add the Poloxamer 407 and mix until dissolved.

3. To 5% of the purified water add the phosphoric acid and monobasic sodium phosphate and mix until dissolved.

4. Add the solution from Step 3 to the solution from Step 2 and mix until homogeneous.

5. Add the benzyl alcohol and mix until homogenous.

6. Bring to final weight with the purified water and mix until the product reaches room temperature.

b. Cold Method

1. Cool 90% of the purified water and maintain its temperature between 4° and 10°C.

Steps 2. through 6. are identical to those used in the Hot Method.

Using either method of manufacture, the formulation is a gel at room temperature.

## EXAMPLE 2

### Preparation of Alpha-2 Interferon Gel Formulation

Add an appropriate quantity of the gel vehicle (prepared as in Example 1) from the tube with flexible walls at approximately 10°C. (liquid state) to the vial containing the lyophilized alpha-2 interferon formulation (containing the required interferon dose and having a specific activity of at least $5 \times 10^7$ International units/mg total protein). Gently shake the vial and bring its contents to room temperature, whereupon an elegant gel suitable for topical application is formed.

## EXAMPLE 3

Proceed as Example 1 but first reconstitute the lyophilized interferon formulation with one ml of water by adding the water to the vial. The interferon solution is then added to the tube with flexible walls containing 9 grams of the dermatologically acceptable vehicle, previously cooled to approximately10°C. Gently shake the tube and bring it to room temperature whereupon the solution forms a gel in the tube with flexible walls.

Likewise by following the procedures outlined in Example 1 through 3 alpha interferon gel formulations may be prepared by substituting for 200 mg of Poloxamer 407

a) 150 mg of Poloxamer 407,
b) 400 mg of Poloxamer 407,
c) 300 mg of Poloxamer 335,
d) 600 mg of Poloxamer 235, or
e) 400 mg of Poloxamer 238.

EXAMPLE 4

Similarly, prepare gels of other alpha interferons by substituting alpha-1 or other alpha-type interferon for alpha-2 interferon in the above Examples.

The formulations provided by the kits of the present invention may be used in the topical treatment of viral skin conditions, e.g., herpes. It is understood, however, that the specific dose level for any particular patient will depend upon a variety of factors including the general health, sex, time of administration and severity of the condition undergoing therapy. Generally, the formulations are applied to the infected area 2 to 4 times daily until no further improvement in the patient's condition is noted.

CLAIMS

1. A kit for formulating and dispensing an alpha-type interferon gel composition comprising a) a vial having an open end aseptically sealed and containing about $1 \times 10^4$ to $5 \times 10^8$ International Units of lyophilized alpha-type interferon formulation prepared from an alpha-type interferon having a specific activity of at least $5 \times 10^7$ International Units/mg. total protein; and b) a tube having flexible walls and sealing means at its open end and containing a dermatologically acceptable vehicle which contains a compatible preservative and a sufficient amount of polyoxyethylene polyoxypropylene block polymer for the vehicle to be liquid at about 15°C. and below and which together with the lyophilized alpha-type interferon gels at 15°C. and above.

2. A kit for formulating and dispensing an alpha-type interferon gel composition comprising a) a vial having an open end aseptically sealed and containing lyophilized alpha-type interferon formulation prepared from an alpha-type interferon having a specific activity of at least $5 \times 10^7$ International Units/mg. total protein; and b) a tube having flexible walls and sealing means at its open end and containing a dermatologically acceptable vehicle which contains a compatible preservative and a sufficient amount of polyoxyethylene polyoxypropylene block polymer for the vehicle to be liquid at about 15°C. and below and which together with the lyophilized alpha-type interferon gels at 15°C. and above;

said vial containing about $1 \times 10^4$ to $5 \times 10^8$ International Units of lyophilized alpha-type interferon per gram of vehicle in said tube.

3. A kit as claimed in claim 1 or claim 2 wherein the lyophilized alpha-type interferon is alpha-2 interferon.

4. A kit as claimed in any of claims 1 to 3 wherein the compatible preservative employed in the dermatologically acceptable vehicle is benzyl alcohol.

5. A kit as claimed in any of claims 1 to 4 wherein the dermatologically acceptable vehicle additionally contains a compatible buffer system to maintain the pH of the reconstituted composition from pH 2 to pH 7;

6. A kit as claimed in claim 5 wherein the buffer maintains the pH at 3.0 to 4.5.

7. A kit as claimed in claim 6 wherein the buffer is phosphoric acid/monobasic sodium phosphate.

8. A kit as claimed in any of claims 1 to 7 wherein the dermatologically acceptable vehicle contains 0.1 to 1.0 percent by weight titanium dioxide.

9. A method for preparing an alpha-type interferon gel composition comprising:

a) dissolving lyophilized alpha-type interferon in a vial by adding a measured amount of distilled water;

b) adding the resulting solution of alpha-type interferon to a tube with flexible walls containing a dermatologically acceptable vehicle maintained at about 15°C. or below, said vehicle containing a compatible preservative and a sufficient amount of polyoxyethylene polyoxypropylene block polymer for the vehicle to be liquid at about 15°C. or below and to gel at above about 15°C.; and

c) forming the gel from the said vehicle and alpha-type interferon solution by allowing the contents of the tube to reach a temperature above about 15°C.

10. A method for preparing an alpha-type interferon gel composition comprising:

a) dissolving lyophilized alpha-type interferon at about 15°C. or below in a measured amount of a dermatologically acceptable vehicle which contains a compatible preservative and a sufficient amount of polyoxyethylene polyoxypropylene block polymer for the vehicle to be liquid at about 15°C. and below and which together with the lyophilized alpha-type interferon gels at 15°C. and above;

b) transferring the reconstituted solution of interferon to a tube with flexible walls; and

c) forming the gel from the said vehicle and alpha-type interferon solution by allowing the contents of the tube to reach a temperature above about 15°C.

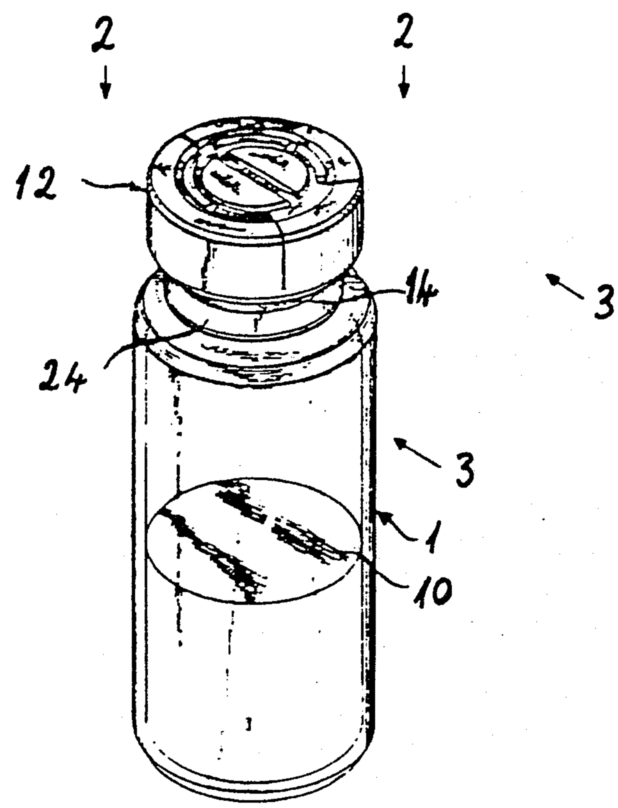


FIG. 1

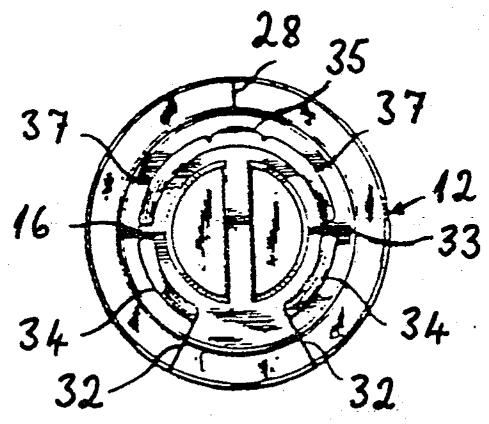


FIG. 2

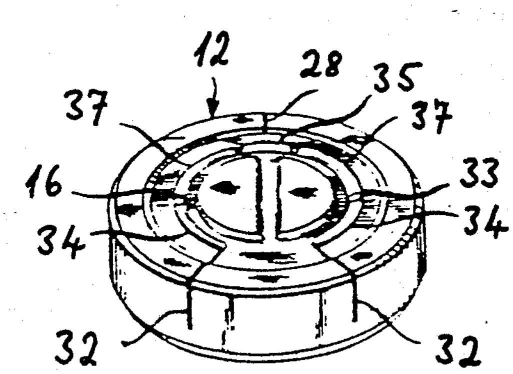


FIG. 3

0127130

FIG. 4

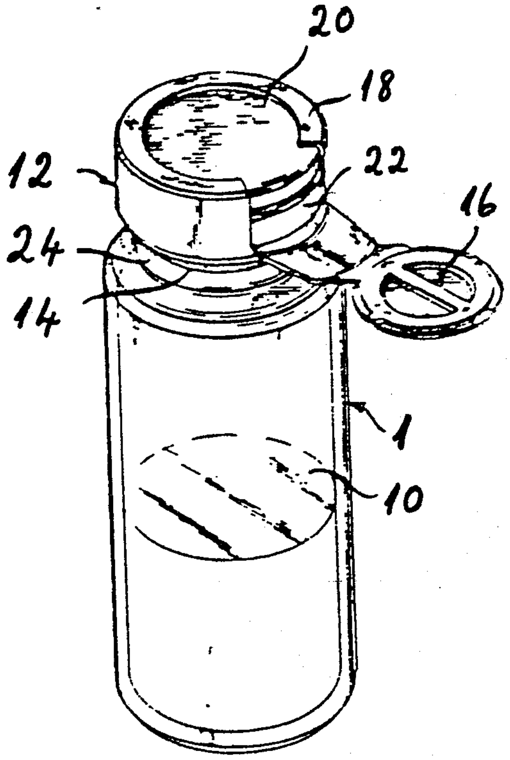


FIG. 5

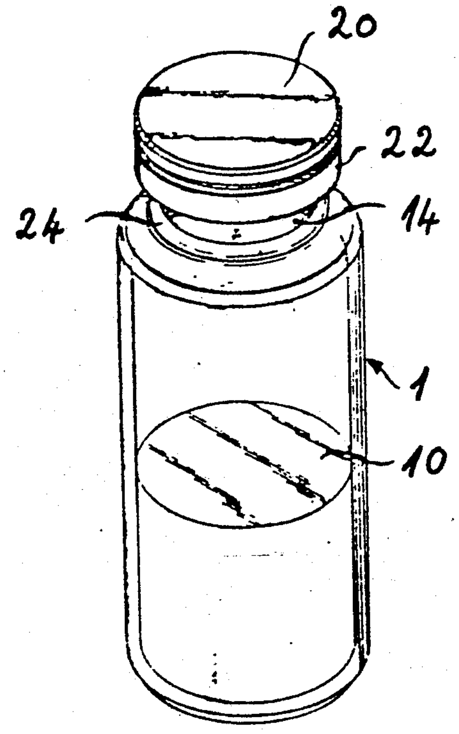


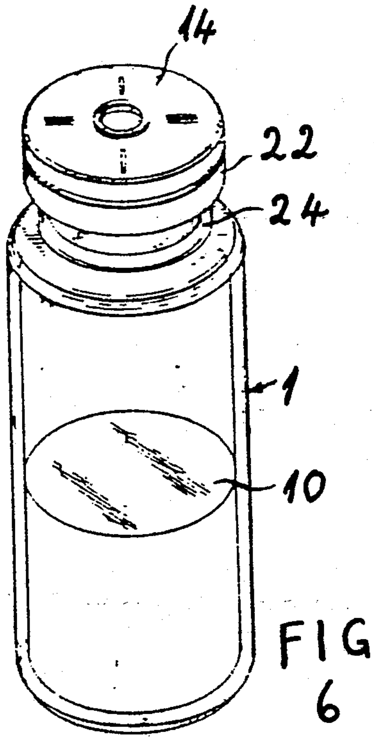


FIG. 6

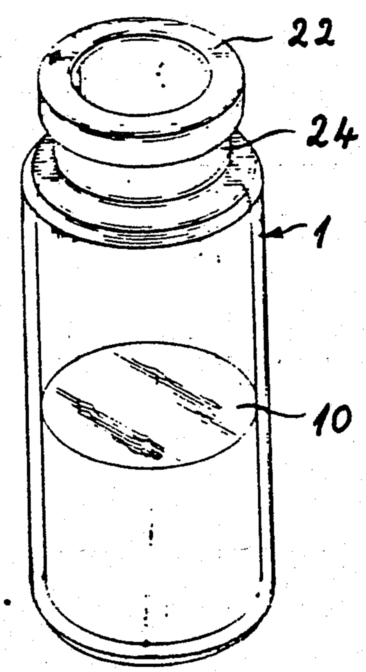


FIG. 7

FIG. 8

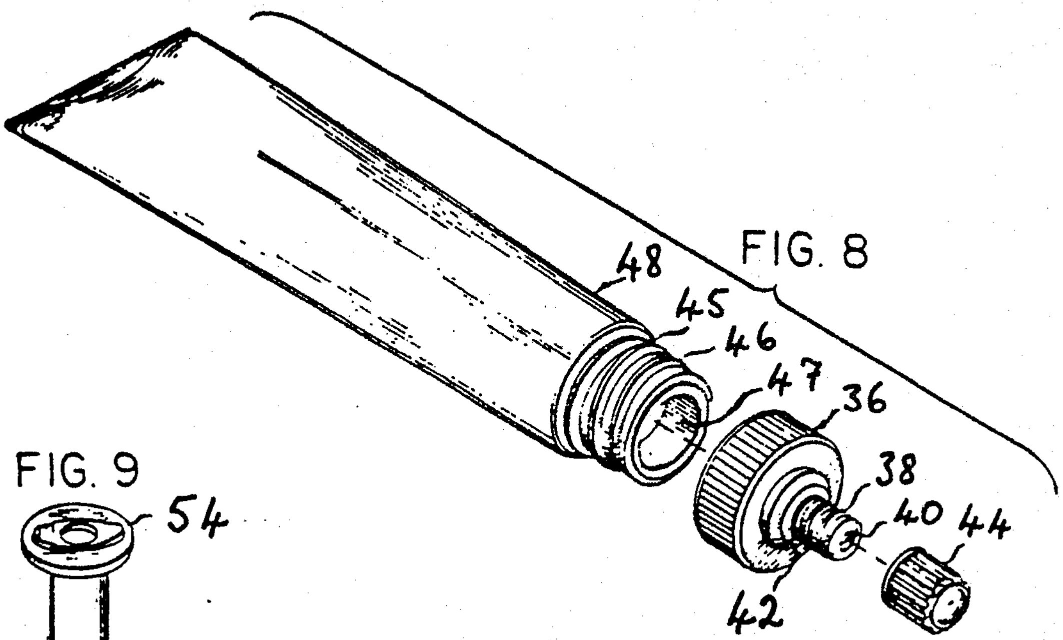


FIG. 9

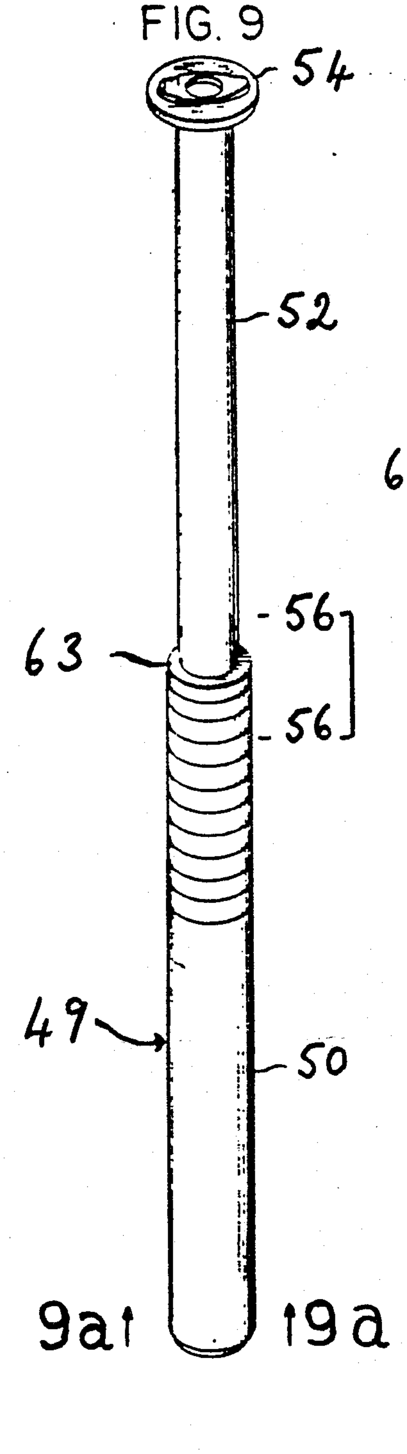


FIG. 9a

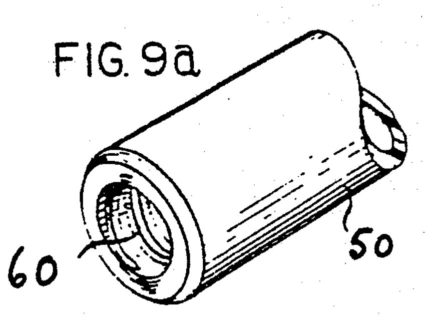


FIG. 10

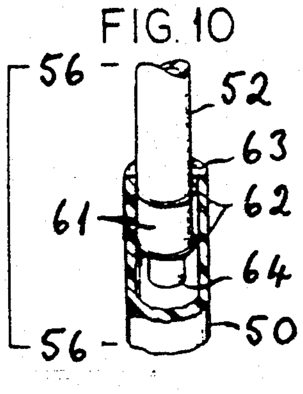


FIG. 11

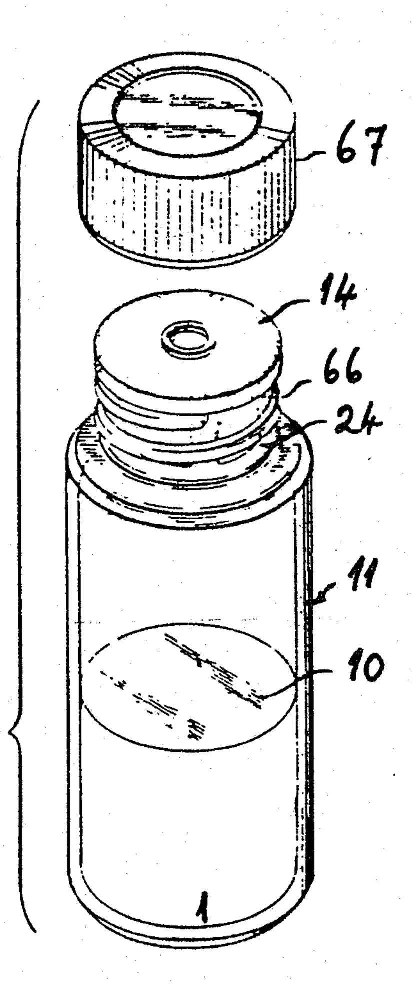

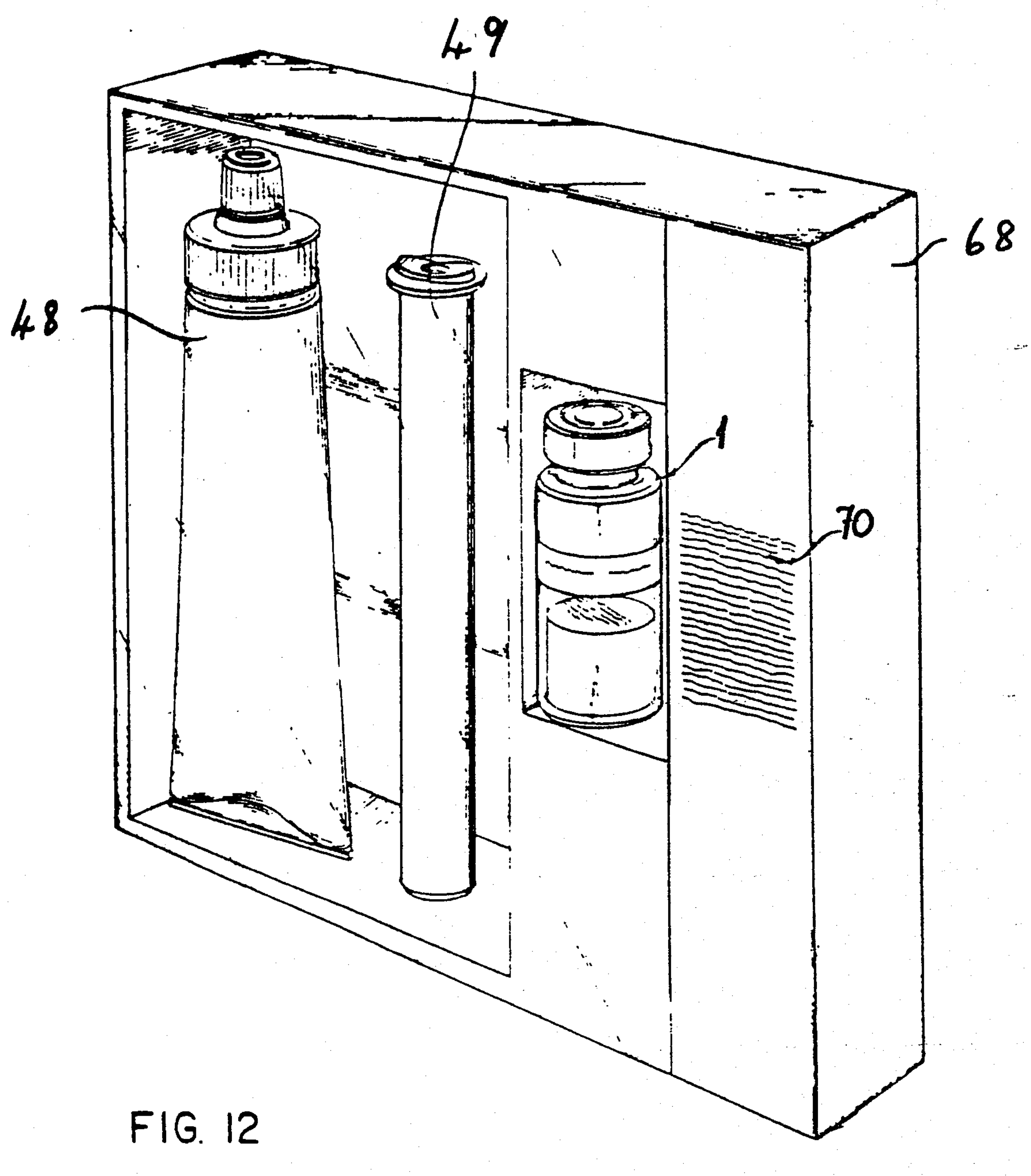


FIG. 12